# EUROPÄISCHE PATENTSCHRIFT

(11) **EP 1 532 263 B1**
(45) Veröffentlichungstag und Bekanntmachung des Hinweises auf die Patenterteilung: **11.10.2006**
(21) Anmeldenummer: 03779784.2
(22) Anmeldetag: 20.08.2003
(51) Int. Cl.: C12P 23/00, A23K 1/00, C12N 9/18

(54) **VERFAHREN ZUR HYDROLYSE VON CAROTINOIDESTERN**
METHOD FOR HYDROLYSING CAROTENOIDS ESTERS
PROCEDE POUR L'HYDROLYSE D'ESTERS DE CAROTENOIDES

(30) Priorität: 20.08.2002 DE 10238980; 13.11.2002 DE 10253112; 16.12.2002 DE 10258971
(43) Veröffentlichungstag der Anmeldung: 25.05.2005
(73) Patentinhaber: Sungene GmbH & Co. KGaA, 06466 Gatersleben (DE)
(72) Erfinder: FLACHMANN, Ralf, 06484 Quedlinburg (DE); SCHOPFER, Christel, 06484 Quedlinburg (DE); SAUER, Matt, 06484 Quedlinburg (DE); KLEBSATTEL, Martin, 06484 Quedlinburg (DE)
(74) Vertreter: Reitstötter - Kinzebach
(86) Internationale Anmeldenummer: PCT/EP2003/009218
(87) Internationale Veröffentlichungsnummer: WO 2004/022765

(56) Entgegenhaltungen:
- EP-A- 1 398 373
- WO-A-02/094982
- ZORN H ET AL: "Enzymatic hydrolysis of carotenoid esters of marigold flowers (tagetes erecta L.) and red paprika (capsicum annuum L.) by commercial lipases and pleurotus sapidus extracellular lipase" ENZYME AND MICROBIAL TECHNOLOGY, STONEHAM, MA, US, Bd. 32, 8. April 2003 (2003-04-08), Seiten 623-628, XP002971542 ISSN: 0141-0229
- BREITHAUPT D E: "ENZYMATIC HYDROLYSIS OF CAROTENOID FATTY ACID ESTERS OF RED PEPPER (CAPSICUM ANNUUM L.) BY A LIPASE FROM CANDIDA RUGOSA" ZEITSCHRIFT FUER NATURFORSCHUNG. C, A JOURNAL OF BIOSCIENCES, TUEBINGEN, DE, Bd. 55, Nr. 11-1, 2000, Seiten 971-975, XP008013161 ISSN: 0939-5075 in der Anmeldung erwähnt
- BREITHAUPT DIETMAR E ET AL: "Carotenol fatty acid esters: Easy substrates for digestive enzymes?" COMPARATIVE BIOCHEMISTRY AND PHYSIOLOGY PART B BIOCHEMISTRY AND, Bd. 132B, Nr. 4, August 2002 (2002-08), Seiten 721-728, XP001188945 August, 2002 ISSN: 1096-4959
- T. AAKERMANN ET AL.: "Enzymatic Hydrolysis of Esters of Alkali labile Carotenols" BIOCATALYSIS AND BIOTRANSFORMATION, Bd. 13, 1996, Seiten 157-163, XP008029126 OPA (OVERSEAS PUBLISHERS ASSOCIATION) AMSTERDAM B.V. PUBLISHED IN THE NETHERLANDS BY HARWOOD ACADEMIC PUBLISHERS GMBH in der Anmeldung erwähnt
- P.B. JACOBS ET AL.: "The cleavage of Carotenoid esters by cholesterol Esterase" COMP. BIOCHEM. PHYSIOL., Bd. 72B, 1982, Seiten 157-160, XP008029124 PERGAMON PRESS LTD. in der Anmeldung erwähnt

## Beschreibung

Die vorliegende Erfindung betrifft ein Verfahren zur enzymatischen Hydrolyse von Carotinoidestern, sowie die Verwendung der auf diese Weise hergestellten Carotinoide als Nahrungs- und Futtermittel.

Carotinoide werden de novo in Bakterien, Algen, Pilzen und Pflanzen synthetisiert. Ketocarotinoide, also Carotinoide, die mindestens eine Keto-Gruppe enthalten, wie beispielsweise Astaxanthin, Canthaxanthin, Echinenon, 3-Hydroxyechinenon, 3'-Hydroxyechinenon, Adonirubin und Adonixanthin, sind natürliche Antioxidantien und Pigmente, die von einigen Algen und Mikroorganismen als Sekundärmetabolite produziert werden.

So wird beispielsweise Astaxanthin von Bakterien und Hefe synthetisiert. Diese akkumulieren freies, nicht verestertes Astaxanthin. Mikroalgen marinen Ursprungs synthetisieren Astaxanthin und akkumulieren dieses in der Regel als Astaxanthinester, geringe Mengen an freiem Astaxanthin sind jedoch auch nachweisbar. Das bekannteste Beispiel ist *Haematococcus pluvialis,* eine Alge, die unter Licht- und Mineralienstress innerhalb von wenigen Tagen bis zu 5% des Trockengewichtes als Astaxanthinester (70-80% als Monoester) akkumuliert. Diese Algenanzucht unter Stressbedingungen wird großtechnisch und wirtschaftlich genutzt.

Astaxanthinester, hauptsächlich Diester, werden im Pflanzenreich natürlicherweise nur von einer Pflanze, dem Adonisröschen, produziert. Die Astaxanthinester werden in den Blütenblättern angereichert; freies Astaxanthin ist lediglich in äußerst geringen Spuren nachweisbar.

In genetisch verändertem Tabak (Mann, Harker, Pecker und Hirschberg; Nature Biotechnology 18(8), 888-892, 2000) wurde in den Nektarien der Blüte Astaxanthin synthetisiert. Die überwiegende Menge an Astaxanthin wird als Ester gespeichert.

Aufgrund ihrer farbgebenden Eigenschaften werden die Ketocarotinoide und insbesondere Astaxanthin als Pigmentierhilfsstoffe in der Tierernährung, vor allem in der Forellen-, Lachs- und Shrimpszucht verwendet.

Zur Freisetzung der Carotinoide aus ihren Estern werden diese in herkömmlicher Weise in ein organisches Lösungsmittel überführt und anschließend alkalisch verseift. Dabei sind aber insbesondere Ketocarotinoide, wie Astaxanthin, Hydroxyechinenone, Adonirubin und Adonixanthin, sehr oxidationsempfindlich, wodurch die Ausbeute an Wertprodukt stark vermindert wird.

Aus dem Stand der Technik ist auch die enzymatische Esterspaltung, insbesondere von Carotinoidestern, unter Verwendung von Lipasen oder Esterasen bekannt.

So beschreiben z.B. Liu et al in J. Nutritional Biochemistry 9, 178-183 (1998) die Lipasekatalysierte Hydrolyse von Xanthophyllen in Gegenwart von Gallensäure. Es wurden aber keine Ketocarotinoide analysiert.

Breithaupt beschreibt in Z. Naturforsch., 55c, 971-975 (2000) die enzymatische Hydrolyse von Carotinoidestern aus Roter Paprika mit Lipase aus Candida rugosa. Experimentelle Untersuchungen mit oben beschriebenen Ketocarotinoidestem werden nicht beschrieben. Für die untersuchten Carotinoidester werden Hydrolyseraten von 21 bis 59 % beschrieben.

Zom et al. beschreiben in Enzyme and Microbial Technology, 32, 623 - 628 (2003) die Hydrolyse von Carotinoidestern aus Tagetes erecta und Capsicum annuum. Hydrolyseraten von 18 bis 44 % werden für Tagetes erecta und 30 bis 69% für Capsicum annuum beschrieben. Die Hydrolyse von oben beschriebenen Ketocarotinoidestern wurde nicht untersucht.

Aakermann et al. beschreiben in Biocatalysis and Biotransformation, 13, 157-163 (1996) die praktisch gescheiterten Versuche zur Hydrolyse von synthetischem Asthaxanthin-Dipalmitat mit Lipase aus Candida rugosa. Es wurden lediglich 5% freies Astaxanthin und 6% Monopalmitat nachgewiesen. Auch mit Astaxanthin-Diacetat wurden keine signifikant besseren Ergebnisse erzielt.

Esterase-katalysierte Spaltungen von Carotinoidestern werden von Jacobs et al. in Comp. Biochem. Physiol. 72B, 157-160 (1982) beschrieben. Astaxanthin-Diester aus Procambarus acutus (Flußkrebs) werden zu 31% in den Monoester und zu 13% in freies Astaxanthin überführt. Eine Hydrolyse von bis zu 85% wird durch Erhöhung der Cholesterin-Esterase Konzentration für möglich gehalten, wobei aber jeglicher experimentelle Beleg für diese Behauptung fehlt. Die Autoren führen außerdem aus, dass die Hydrolyse von der Art der Fettsäuren im Ester beeinflusst werden kann, ohne jedoch nähere Angaben zu machen.

Die enzymatischen Methoden zur Carotinoidesterhydrolyse gemäß Stand der Technik führen somit insgesamt zu keinen völlig zufriedenstellenden Ergebnissen, da die tatsächlich erzielten Hydrolyseraten sehr gering sind. Enzymatische Ketocarotinoidester-Hydrolysen gemäß Stand der Technik verlaufen, soweit untersucht, ebenfalls noch nicht zufriedenstellend. Verlässliche Aussagen über den möglichen Verlauf der Hydrolyse-Reaktion bei Verwendung anderer Carotinoidester-Quellen, wie insbesondere Algen und Pflanzen, sind daher für den Fachmann nicht möglich.

### Kurze Beschreibung der Erfindung

Aufgabe der vorliegenden Erfindung ist daher die Bereitstellung eines verbesserten Verfahrens zur enzymatischen Hydrolyse von Carotinoidestem und insbesondere Ketocarotinoidestem.

Obige Aufgabe wurde gelöst durch Bereitstellung eines Verfahrens zur enzymatischen Hydrolyse von Carotinoidestern, wobei man einen Carotinoidester-haltigen Reaktanden, abgeleitet von einem natürlichen oder genetisch verändertem Organismus, mit einem esterspaltenden Enzym, ausgewählt unter Carboxylsäureester-spaltenden Hydrolasen (E.C. 3.1.1.), bis zur im wesentlichen quantitativen hydrolytischen Esterspaltung inkubiert und das (die) gebildete(n) Carotinoid(e) gegebenenfalls aus dem Reaktionsansatz isoliert.

### Detaillierte Beschreibung der Erfindung

### a) allgemeine Definitionen:

In Rahmen der vorliegender Erfindung sind "Carotinoide" ausgewählt unter veresterbaren Carotinoiden, wie Zeaxanthin, alpha-Cryptoxanthin, beta-Cryptoxanthin, Capsorubin, Capsanthin, Violaxanthin, Neoxanthin, Lutein, Astaxanthin, Adonixanthin, Adonirubin, 3'-Hydroxyechinenon, 3-Hydroxyechinenon, ohne darauf beschränkt zu sein.

Eine bevorzugte Gruppe von Carotinoiden sind Ketocarotinoide.

In Rahmen der vorliegender Erfindung sind "Ketocarotinoide" ausgewählt unter veresterbaren, ketogruppenhaltigen Verbindungen, wie Astaxanthin, 3-Hydroxyechinenon, 3'-Hydroxyechinenon, Adonirubin und Adonixanthin sowie Gemischen dieser Verbindungen.

Der "Ester" eines Carotinoids/Ketocarotinoids kann sowohl ein Mono- als auch Polyester, insbesondere Diester oder ein Gemisch von verschiedenen Estern sein. Di- oder Polyester können von gleichen oder verschiedenen Carbonsäuren abgeleitet sein.

Ester sind insbesondere Ester von Fettsäuren. Geeignete Fettsäureester sind aufgebaut aus geradkettigen oder verzweigten, ein- oder mehrfach ungesättigten, gegebenenfalls substituierten C₈-C₃₀-Monocarbonsäuren. Beispiele für gesättigte unverzweigte Fettsäuren sind Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure. Beispiele für einfach ungesättigte Fettsäuren sind Palmitoleinsäure, Ölsäure und Erucasäure. Beispiele für zweifach ungesättigte Fettsäuren sind Sorbinsäure und Linolsäure. Beispiele für dreifach ungesättigte Fettsäuren sind Linolensäure und Elaeostearinsäure. Beispiele für vier- und mehrfach ungesättigte Fettsäuren sind Arachidonsäure, Clupanodonsäure und Docosahexaensäure. Bevorzugt sind unverzweigte, gesättigte Fettsäuren. Weiterhin bevorzugt sind einwertige, gesättigte oder eine, zwei- oder dreifach ungesättigte C₁₀₋₂₄-, vorzugsweise C₁₂₋₂₀- oder C₁₄₋₂₀-Fettsäuren.

Carotinoide bzw. Ester gemäß obiger Definition umfassen diese Verbindungen sowohl in isomerenreiner Form als auch in Form von Stereisomerengemischen.

"Gesamtcarotinoide" steht für die Summe aller Carotinoide und Carotinoidester gemäß obiger Definition.

Eine "im wesentlichen quantitative hydrolytische Esterspaltung" bedeutet, dass erfindungsgemäß durch enzymatische Aktivität wenigstens einer der vorliegenden Carotinoidester, insbesondere wenigstens einer der vorliegenden Ketocarotinoidester, zu wenigstens etwa 85 %, insbesondere wenigstens etwa 88 % hydrolysiert ist, so dass keine Estergruppen mehr im Molekül enthalten sind. Besonders bevorzugt erhält man erfindungsgemäß Hydrolyseraten von 100% oder weniger, wie z.B. 88 bis 99% oder 95 bis 99%.

Die "Hydrolyserate" ist erfindungsgemäß definiert durch den prozentualen Anteil, um den die Menge an (z.B. extrahierten) Carotinoidestern in einem Reaktanden enzymkatalysiert abnimmt. Insbesondere lässt sich die Hydrolyserate dadurch bestimmen, dass man den Carotinoidester-Gehalt im Reaktanden vor und nach der erfindungsgemäßen enzymatischen Behandlung, z.B. chromatographisch wie in den Beispielen beschrieben, bestimmt und daraus den Anteil hydrolysierter Ester bestimmt.

Der im Verfahren eingesetzte esterhaltige "Reaktand" kann von natürlichen oder genetisch veränderten, rekombinanten Organismen abgeleitet sein. Der Reaktand kann dabei aus dem gesamten Organismus oder einem Teil (z.B. Blütenblättern) oder einer Fraktion (z.B. erhalten nach Homogenisierung oder Zellaufschluss und anschließende Fraktionierung) davon stammen.

Der "Carotinoidgehalt" eines Reaktanden ist definiert als die Menge an Gesamtcarotinoiden, bestimmt durch photometrische Messung nach der Methode von Lichtenthaler (Methods Enzymology 148, 350-382, 1987).

Der "Carotinoidestergehalt" ist definiert als die Menge an Carotinoiden, die mit einer gesättigten oder ein-, zwei- oder dreifach ungesättigten C₁₀₋₂₄-, vorzugsweise C₁₂₋₂₀₋oder C₁₄₋₂₀-Monocarbonsäure verestert sind. Der Gehalt an Carotinoidestern kann unter anderem chromatographisch ermittelt werden, da Carotinoidester bei geeigneten "reverse phase"-Trägermaterialien, wie z.B. langkettigen polymergebundenen C30-Phasen in der Regel längere Retentionszeiten aufweisen als ungebundene Carotinoide. Ein geeignetes C30 Trägermaterial und geeignete Trennbedingungen sind beispielhaft in Beispiel 3 genannt.

Eine enzymatische Einheit (U) Lipase bedeutet diejenige Menge an Enzym, die 1,0 Microäquivalente Fettsäure aus einen Triglycerid (z.B.Glycerintripalmitat) in einer Stunde bei 37°C und pH 7,7 freisetzt.

Eine enzymatische Einheit (U) Esterase bedeutet diejenige Menge an Enzym, welche innerhalb einer Minute 1,0 Micromol Ester-Substrat bei pH 7,0 und 37°C hydrolytisch vollständig spaltet. 1 U Cholesterol Esterase bedeutet so z.B. diejenige Menge an Enzym, die unter den angegebenen Bedingungen 1 Micromol Cholesteryloleat in Gegenwart von Taurocholat zu Cholesterin und Oleinsäure innerhalb von einer Minute umsetzt.

"Organismen" umfassen erfindungsgemäß pro- und eukaryotischen Mikroorganismen oder Pflanzen.

"Mikroorganismen" sind dabei ausgewählt unter Bakterien, Hefen, Algen oder Pilzen.

Bevorzugte Mikroorganismen sind ausgewählt unter *Escherichia, Erwinia, Agrobacterium, Flavobacterium, Alcaligenes, Bacillus, Paracoccus, Nostoc,* Cyanobakterien der Gattung *Synechocystis, Candida, Saccharomyces, Hansenula, Halobacterium, Phaffia, Pichia, Aspergillus, Trichoderma, Ashbya, Neurospora, Blakeslea, Phycomyces, Fusarium, Haematococcus, Phaedactylum tricomatum, Volvox, Chlamydomonas, Ankistrodesmus, Chlorella, Chlorococcum, Coelastrum, Crucigenia, Dictyococcus, Hydrodicty on, Ermosphera, Coelastrella, Botryococcus, Scenedesmus, Scotiellopsis, Protosiphon, Euglena, Nannochloropsis, Glenodinium, Aphanocapsa, Parmotrema, Cantharellus, Brevibacterium, Eremosphera, Pavlova, Gordonia, Isochryis, Brydyrhizobium, Myrmecia, Neochloris, Mycobacterium* oder *Dunaliella.*

Erfindungsgemäß geeignete Pflanze sind ausgewählt aus den Familien Ranunculaceae, Berberidaceae, Begoniaceae, Papaveraceae, Cannabaceae, Chenopodiaceae, Cruciferae, Rosaceae, Fabaceae, Linaceae, Vitaceae, Brassiceae, Cucurbitaceae, Primulaceae, Caryophyllaceae, Amaranthaceae, Apocynaceae, Balsaminaceae, Gentianaceae, Geraniaceae, Graminae, Euphorbiaceae, Labiatae, Leguminosae, Caprifoliaceae, Oleaceae, Tropaeolaceae, Solanaceae, Lobeliaceae, Scrophulariaceae, Compositae, Asteraceae, Plumbaginaceae, Liliaceae, Amaryllidaceae, Rubiaceae, Poaceae, Polemoniaceae, Orchidaceae, Umbelliferae, Verbenaceae, Violaceae, Malvaceae, Illiaceae oder Lamiaceae.

Insbesondere sind Pflanzengattungen wie Marigold, Tagetes erecta, Tagetes patula, Acacia, Aconitum, Adonis, Amica, Aquilegia, Aster, Astragalus, Bignonia, Calendula, Caltha, Campanula, Canna, Centaurea, Cheiranthus, Chrysanthemum, Citrus, Crepis, Crocus, Curcurbita, Cytisus, Delonia, Delphinium, Dianthus, Dimorphotheca, Doronicum, Eschscholtzia, Forsythia, Fremontia, Gazania, Gelsemium, Genista, Gentiana, Geranium, Gerbera, Geum, Grevillea, Helenium, Helianthus, Hepatica, Heracleum, Hibiscus, Heliopsis, Hypericum, Hypochoeris, Impatiens, Iris, Jacaranda, Kenia, Laburnum, Lathyrus, Leontodon, Lilium, Linum, Lotus, Lycopersicon, Lysimachia, Maratia, Medicago, Mimulus, Narcissus, Oenothera, Osmanthus, Petunia, Photinia, Physalis, Phyteuma, Potentilla, Pyracantha, Ranunculus, Rhododendron, Rosa, Rudbeckia, Senecio, Silene, Silphium, Sinapsis, Sorbus, Spartium, Tecoma, Torenia, Tragopogon, Trollius, Tropaeolum, Tulipa, Tussilago, Ulex, Viola und Zinnia geeignet.

### b) Beschreibung bevorzugter Ausführungsformen

Ein erster Gegenstand der Erfindung betrifft ein Verfahren zur enzymatischen Hydrolyse von Ketocarotinoidestem gemäß der Definition in Anspruch 1 oder 2.

In einer bevorzugten Variante des Verfahrens liegt die Reaktionszeit im Bereich von mehr als 1 Stunde, wie z.B. 1 bis 48, 5 bis 45,10 bis 40, 15 bis 35 oder 20 bis 30 Stunden. Die erforderliche Länge der Reaktion bis zur Erzielung der gewünschten im wesentlichen quantitativen Hydrolyse kann vom Fachmann durch Probenahme und Analyse z.B. der verbrauchten Estermenge, in einfacher Weise bestimmt werden.

Erfindungsgemäß bevorzugt eingesetzte Hydrolasen sind ausgewählt unter Lipasen (E.C. 3.1.1.3) und Esterasen (E.C. 3.1.1.13), sowie Gemischen davon. Bevorzugte Enzyme sind z.B. Lipasen aus Candida sp., wie z.B. Lipase Typ 7 aus Candida rugosa oder eine Cholesterin Esterase aus Pseudomonas fluorescens und Pseudomonas spec., Rinderpankreas und Schweinepankreas. Weitere bevorzugte Lipasen sind bekannt aus Candida antarctica, Candida cylindrica, Candida cylindracea, Candida lipolytica, Pseudomonas cepacia, Pseudomonas spec., Pseudomonas fluorescens, Pseudomonas alcaligenes, Schweinepankreas, Schweineleber, Humicola spec., Rhizomucor miehei, Rhizomucor javanicus, Rhizopus arrhizus, Rhizopus japanicus, Rhizopus niveus, Rhizopus delemar, Alcaligenes spec., Penicillium roqueforti, Penicillium cyclopium, Carica papaya L., Mucor miehei, Aspergillus niger, Chromobacter viscosum, Thermomyces lanuginosa. Die erfindungsgemäßen Enzyme können dabei in freier (z.B. gelöster) oder immobilisierter, trägergebundener Form vorliegen. Methoden zur Enzym-Immobilisierung sind dem Fachmann geläufig und z.B. in Biotechnology, Rehm et al Hrsg., Vol.3 VCH Verlagsgesellschaft, 2. Auflage, Kapitel 17 beschrieben.

Insbesondere ist es bevorzugt, dem Reaktionsansatz das Enzym in einer solchen Gesamtmenge zugesetzt wird, dass die Gesamtaktivität an zugesetztem Enzym, jeweils bezogen auf den Gesamtcarotinoid-Gehalt (in µg bzw. mg) in folgendem Bereich liegt:
- Lipase: mehr als 10, insbesondere mehr als 20, oder mehr als 30, wie insbesondere 50 bis 5.000 oder 50 bis 4.000 oder 50 bis 3.000 oder 500 bis 3.000 U/µg Gesamtcarotinoide.
- Esterase: mehr als 1, insbesondere mehr als 5, wie z.B. 10 bis 500 oder 50 bis 300 oder 100 bis 200 U/mg Gesamtcarotinoide.

Die angegebenen Gesamtenzymaktivitäten entsprechen der bei Reaktionsbeginn vorgelegten Enzymaktivität, oder, falls die Zugabe in mehreren Portionen zu verschiedenen Zeiten der Reaktion erfolgen sollte, der rechnerischen Summe der anfänglichen Enzymaktivitäten der zugesetzten Einzelportionen.

In einer bevorzugten Variante des Verfahrens wird der Carotinoidester-haltige Reaktand in einem wasserhaltigen Reaktionsmedium mit dem esterspaltenden Enzym inkubiert, wobei die Carotinoidester gegebenenfalls in emulgierter Form im Medium vorliegen und wobei dem Reaktionsmedium gegebenenfalls wenigstens ein Emulgator in einer Menge zugesetzt wird, so dass das Mengen-Verhältnis von Emulgator (z.B. in mg) zu Carotinoid (z.B. in mg) im Bereich von etwa 100:1 bis 2000:1, wie etwa in Bereich von 500:1 bis 1000:1 liegt.

Vorzugsweise umfasst der Emulgator wenigstens eine Verbindung, ausgewählt unter Cholansäure und Derivaten davon sowie Gemischen dieser Verbindungen. Insbesondere ist der Emulgator eine (vorzugsweise in etwa äquimolare) Mischung von Cholsäure und Desoxycholsäure ist.

Zusätzlich oder anstelle des oben genannten Emulgatorsystems können im erfindungsgemäßen wässrigen Reaktanden andere die Löslichkeit hydrophober Carotinoidester verbessernder Zusätze enthalten sein. Beispiele hierfür sind Detergentien, wie z.B. Digitonin, Octylglucosid, Brij 35 (Dodecylpoly(oxyethylenglycolether)), Genapol X-080 (Isotridecyl(ethylenglycolether)), Nonidet P-40 (Ethylphenolpoly(ethylenglycolether)), Synperonic P/F 68 (Polyethylenglycol-polypropylenglycol-copolymer), Synperonic P/F 127 (Polyethylenglycol-polypropylenglycol-copolymer), Thesit (Dodecylpoly(ethylenglycolether), Triton X-100 und Triton X-114 (Octylphenolpoly(ethylenglycolether), Tween 20 und Tween 80 (Poly(oxyethylene) sorbitanmonooleate), Decanoyl-N-methylglucamid (MEGA 10; N-(D-Gluco-2,3,4,5,6-pentahydroxyhexyl)-N-methyldecanamid), Deoxy-BigCHAP (N,N-bis-(3-D-gluconamidopropyl)-deoxycholamid), n-Dodecylglucosid (1-O-n-Dodecyl-β-D-glucopyranosid), n-Dodecylmaltosid (1-O-n-Dodecyl-β-D-glucopyranosyl (1→4) α-D-glucopyranosid), HECAMEG (6-O-(N-heptyl-carbamoyl)-methyl-α-D-glucopyranosid), Octanoyl-N-Methylglucamid (MEGA 8; N-(D-Gluco-2,3,4,5,6-pentahydroxyhexyl)-N-methyloctanamid), Sucrosemonolaurat, Taurocholsäure, Taurodeoxycholsäure, Lithiumdodecylsulfat, Natriumdodecylsulfat, CHAPS (3-((3-Cholamidopropyl)dimethylammonio)-1-propan-sulfonat), CHAPSO (3-((3-Cholamidopropyl)dimethylammonio-2-hydroxy-1-propan-sulfonat), N-Dodecyl-N,N-dimethyl-3-ammonio-1-propan-sulfonat (Sulfobetain SB12), N-Tetradecyl-N,N-dimethyl-3-ammonio-1-propan-sulfonat (Sulfobetain SB14) oder Lösungsvermittlern.

Insbesondere kann das Reaktionsmedium ein wässrig-organisches Medium sein, welches ein organisches Lösungsmittel in einem Volumenanteil von etwa 5 bis 95 Vol.-%, wie z.B. 5 bis 50 oder 5 bis 30 oder 5 bis 15 Vol.-%, bezogen auf das Gesamtvolumen des Reaktionsansatzes, enthält. Das organischen Lösungsmittel ist dabei ausgewählt unter niedrig-aliphatischen Ketonen (wie Aceton), C₄-C₁₀-Alkanen (wie n-Butan, n-Pentan, n-Hexan, n-Heptan, n-Octan und die ein- oder mehrfach verzweigten Analoga davon), C₁-C₁₀-Alkanolen (insbesondere Methanol, Ethanol, n- oder iso-Propanol, n-oder tert.-Butanol), Ethern (wie Di-C₂-C₆-alkylethern, wie z.B. Isopropylether), oder DMSO und DMF und aromatischen Lösungsmitteln (wie Benzol, Xylol oder Toluol) und Gemischen davon. Bei Verwendung von Aceton ist ein Volumenanteil des Acetons im Bereich von etwa 5 bis 15 Vol.-% , insbesondere etwa 10 Vol.-%, bevorzugt.

insbesondere wird das erfindungsgemäße Verfahren so durchgeführt, dass der Reaktionsansatz einen pH-Wert im Bereich von etwa 6 bis 8, insbesondere 6,5 bis 7,5, aufweist. Zur Einstellung des pH-Wertes können übliche Puffer, wie Alkaliphosphat-, Tris-, PIPES- oder HEPES-Puffer, verwendet werden. Andere geeignete Puffer sind dem Fachmann geläufig. Die Auswahl von Art, Konzentration und Pufferbereich obliegt dem Fachmann und kann dem jeweils verwendeten Reaktionsansatz durch wenige Vorversuche optimal angepasst werden

In einer weiteren bevorzugten Ausführungsform des erfindungsgemäßen Verfahrens wird der Carotinoidester-haltigen Reaktanden in einem im Wesentlichen nichtwässrigen Reaktionsmedium mit dem esterspaltenden Enzym inkubiert. Das nichtwässrige Reaktionsmedium enthält dabei wenigstens ein organisches Lösungsmittel, ausgewählt unter C₄-C₁₀-Alkanen, C₁-C₁₀-Alkanolen, Di-C₂-C₆-alkylethern und aromatischen Lösungsmitteln, insbesondere solche gemäß obiger Definition.

Das erfindungsgemäße Verfahren eignet sich besonders zur Hydrolyse eines Reaktanden, der wenigstens einen Carotinoid-Monoester oder -Diester einer C₁₀₋₂₄-, vorzugsweise C₁₄₋₂₀-Monocarbonsäure gemäß obiger Definition umfasst.

Gemäß einer weiteren bevorzugten Ausgestaltung des erfindungsgemäßen Verfahrens ist der Reaktand abgeleitet aus natürlichen oder rekombinanten, pro- oder eukaryotischen Mikroorganismen oder Pflanzen oder Teilen davon. Der Reaktand wird vorzugsweise durch Extraktion mit Hilfe eines organischen Lösungsmittels (insbesondere gemäß obiger Definition) oder Gemischen von solchen organischen Lösungsmitteln gewonnen.

Gemäß einer weiteren bevorzugten Ausgestaltung des erfindungsgemäßen Verfahrens erfolgt die Reaktion mehrstufig unter wiederholter Zugabe von Enzym. Gewöhnlich ist es ausreichend für eine im Wesentlichen quantitative Esterspaltung, wenn dem Reaktionsmedium in einer zweiten Stufe eine weitere Enzymportion zugesetzt wird. Zeitpunkt und Menge kann der Fachmann in einfacher Weise bestimmen. Stellt er z.B. durch Probennahme fest, dass keine weitere Esterhydrolyse erfolgt, obwohl noch nicht umgesetzter Ester im Medium enthalten ist, so kann er eine ausreichende Menge Enzym nachdosieren. Erforderlichenfalls kann diese Nachdosierung wiederholt werden.

Gemäß einer weiteren bevorzugten Ausgestaltung des erfindungsgemäßen Verfahrens wird das Enzym in trägergebundener Form eingesetzt. Beispiele geeigneter Träger sind z.B. beschrieben in Biotechnology, Vol. 3, a.a.O.)

Die Reaktionstemperatur liegt erfindungsgemäß vorzugsweise im Bereich von etwa 20 bis 70 °C, je nach Temperaturoptimum des eingesetzten Biokatalysators. Beispielsweise kann die Reaktionstemperatur im Bereich von 25 bis 45 oder 30 bis 40 °C liegen.

In einer weiteren Variante umfasst der Carotinoidester-haltige Reaktand wenigstens einen von Ketocarotinoidestem verschiedenen Carotinoidester, wenigstens einen Ketocarotinoidester oder Gemische von solchen Carotinoidestern und Ketocarotinoidestem.

Insbesondere ist der Ketocarotinoidester abgeleitet von Astaxanthin.

Vorzugsweise werden die gespaltenen Carotinoide und/oder Ketocarotinoide in an sich bekannter Weise aus dem Reaktionsmedium isoliert.

Das erfindungsgemäße Verfahren kann batchweise, semi-batchweise oder kontinuierlich betrieben werden. Die Durchführung des Verfahrens kann vorteilhafterweise auch in Bioreaktoren erfolgen, wie z.B. beschrieben in Biotechnology, Vol. 3, (a.a.O.)

Ein anderer Gegenstand der Erfindung betrifft schließlich die Verwendung der in obiger Weise hergestellten Carotinoide zur Herstellung von Nahrungs- und Futtermittelzusätzen.

### c) Herstellung transgener Ketocarotinoidester-produzierender Tagetes-Pflanzen

Es wird eine rekombinante, Ketolase-Aktivität exprimierende Tagetes Pflanze hergestellt. Mit Hilfe dieser Ketolase wird es ermöglicht, in den β-Iononring von Carotinoiden eine Ketogruppe einzuführen. Geeignete Ketolase-kodierende Sequenzen sind aus dem Stand der Technik bekannt, wie z.B. von Haematococcus pluvialis Accession No.: D45881. Andere geeignete Gene und die Herstellung geeigneter Konstrukte sind in der DE 10258971 beschrieben, worauf hiermit ausdrücklich Bezug genommen wird.

### Die Herstellung transgener Pflanzen kann z.B. in folgender Weise erfolgen:

Tagetessamen werden sterilisiert und auf Keimungsmedium (MS-Medium; Murashige and Skoog, Physiol. Plant. 15(1962), 473-497; pH 5.8. 2 % Saccharose) aufgelegt. Die Keimung erfolgt in einem Temperatur/Licht/Zeitintervall von 18 bis 28°C/20-200 µE/3 bis 16 Wochen, bevorzugt jedoch bei 21°C, 20 bis 70 µE, für 4 bis 8 Wochen.

Alle Blätter der sich bis dahin entwickelten in vitro Pflanzen werden geerntet und quer zur Mittelrippe geschnitten. Die dadurch entstehenden Blattexplantate mit einer Größe von 10 bis 60 mm² werden im Verlaufe der Präparation in flüssigem MS-Medium bei Raumtemperatur für maximal 2 h aufbewahrt.

Ein beliebiger Agrobakterium tumefaciens Stamm, bevorzugt aber ein supervirulenter Stamm, wie z.B. EHA105 mit einem entsprechenden Binärplasmid (hergestellt in an sich bekannter Weise), das ein Selektionsmarkergen (bevorzugt *bar* oder *pat)* sowie ein Ketolase-Gen trägt, wird über Nacht angezogen und für die Co-Kultivierung mit dem Blattmaterial verwendet. Die Anzucht des Bakterienstammes kann wie folgt erfolgen: Eine Einzelkolonie des entsprechenden Stammes wird in YEB (0,1 % Hefeextrakt, 0,5 % Rindfleischextrakt, 0,5 % Pepton, 0,5 % Saccharose, 0,5 % Magnesiumsulfat x 7 H₂0) mit 25 mg/l Kanamycin angeimpft und bei 28°C für 16 bis 20 Stunden angezogen. Anschließend wird die Bakteriensuspension durch Zentrifugation bei 6000 g für 10 min geerntet und derart in flüssigem MS Medium resuspendiert, dass sich eine OD₆₀₀ von ca. 0,1 bis 0,8 einstellt. Diese Suspension wird für die Co-Kultivierung mit dem Blattmaterial verwendet.

Unmittelbar vor der Co-Kultivierung wird das MS-Medium, in dem die Blätter aufbewahrt worden sind, durch die Bakteriensuspension ersetzt. Die Inkubation der Blättchen in der Agrobakteriensuspension erfolgt für 30 min unter leichtem Schütteln bei Raumtemperatur. Anschließend werden die infizierten Explantate auf ein mit Agar (z.B. 0,8 % Plant Agar (Duchefa, NL)) verfestigtes MS-Medium mit Wachstumsregulatoren, wie beispielsweise 3 mg/l Benzylaminopurin (BAP) sowie 1 mg/l Indolylessigsäure (IAA) aufgelegt. Die Orientierung der Blätter auf dem Medium ist bedeutungslos. Die Kultivierung der Explantate findet für 1 bis 8 Tage, bevorzugt aber für 6 Tage statt, dabei können folgende Bedingungen angewendet werden: Lichtintensität: 30 bis 80 µMol/m² x sec, Temperatur: 22 bis 24°C, hell/dunkel Wechsel von 16/8 Stunden. Anschließend werden die co-kultivierten Explantate auf frisches MS-Medium, bevorzugt mit den gleichen Wachstumsregulatoren übertragen, wobei dieses zweite Medium zusätzlich ein Antibiotikum zur Unterdrückung des Bakterienwachstums enthält. Timentin in einer Konzentration von 200 bis 500 mg/l ist für diesen Zweck sehr geeignet. Als zweite selektive Komponente wird eine für die Selektion des Transformationserfolges eingesetzt. Phosphinothricin in einer Konzentration von 1 bis 5 mg/l selektiert sehr effizient, aber auch andere selektive Komponenten gemäß des zu verwendenden Verfahrens sind denkbar.

Nach jeweils ein bis drei Wochen erfolgt der Transfer der Explantate auf frisches Medium bis sich Sprossknospen und kleine Sprosse entwickeln, die dann auf das gleiche Basalmediurn einschließlich Timentin und PPT oder alternative Komponenten mit Wachstumsregulatoren, nämlich z.B. 0,5 mg/l Indolylbuttersäure (IBA) und 0,5 mg/l Gibberillinsäure GA₃, zur Bewurzelung übertragen werden. Bewurzelte Sprosse können ins Gewächshaus überführt werden.

Zusätzlich zu der beschriebenen Methode sind folgende vorteilhafte Modifikationen möglich:
- Bevor die Explantate mit den Bakterien infiziert werden, können sie für 1 bis 12 Tage, bevorzugt 3 bis 4, auf das oben beschriebene Medium für die Co-Kultur vorinkubiert werden. Anschließend erfolgt die Infektion, Co-Kultur und selektive Regeneration wie oben beschrieben.
- Der pH Wert für die Regeneration (normalerweise 5,8) kann auf pH 5,2 gesenkt werden. Dadurch wird die Kontrolle des Agrobakterienwachstums verbessert.
- Die Zugabe von AgNO₃ (3 -10 mg/l) zum Regenerationsmedium verbessert den Zustand der Kultur einschließlich der Regeneration selbst.
- Komponenten, die die Phenolbildung reduzieren und dem Fachmann bekannt sind, wie z.B. Citronensäure, Ascorbinsäure, PVP, wirken sich positiv auf die Kultur aus.
- Für das gesamte Verfahren kann auch flüssiges Kulturmedium Verwendung finden. Die Kultur kann auch auf handelsüblichen Trägern, die auf dem flüssigen Medium positioniert werden inkubiert werden.

Aus den auf diese Weise transformierten Pflanzen werden solche mit erhöhter Ketolase-Aktivität selektiert, vermehrt und als Ausgangsmaterial für die erfindungsgemäße Carotinoidester-Hydrolyse eingesetzt.

### d) Extraktionsmethoden für Carotinoidester

Carotinoide und deren Ester, wie Astaxanthin und dessen Mono- und Diester, können aus den Carotinoid-haltigen Mikroorganismen, Pflanzen oder Pflanzenteilen, die gegebenenfalls vorher getrocknet und/oder zerkleinert wurden, durch organische Lösungsmittel extrahiert werden, wie beispielsweise durch Aceton, Hexan, Methylenchlorid, tert.-Butylmethylether oder durch Lösungsmittelgemische wie Ethanol/Hexan oder Aceton/Hexan. Durch unterschiedliche Mischungsverhältnisse der Lösungsmittel kann aufgrund der verschiedenen Polarität die Extraktionswirkung variiert werden. Durch eine solche Extraktion lassen sich Carotinoide und deren Ester in hoher Konzentration anreichem.

Anschließend kann durch Ausschütteln der Carotinoide und deren Ester und chromatografische Auftrennung des Gemisches deren Reinheit weiter erhöht werden.

Auf diese Weise hergestellte Extrakte eignen sich besonders als Reaktand zur Durchführung der erfindungsgemäßen Reaktion.

### e) Allgemeine Durchführung der Lipase-katalysierten Esterhydrolyse

Zerkleinertes Ausgangsmaterial, z.B. gemörsertes Petalenmaterial, wird mit einem organischen Lösungsmittel, z.B.100% Aceton, gegebenenfalls mehrmals über einen geeigneten Zeitraum unter Schütteln (z.B. dreimal, jeweils etwa 15 Minuten) extrahiert. Das Lösungsmittel wird evaporiert. Carotinoide werden anschließend in einem geeignete Volumen organischem Lösungsmittels, z.B. Aceton aufgenommen, mit geeignetem Puffer, z.B. Kaliumphosphat-Puffer (100 mM, pH7.4), verdünnt, z. B. im Verhältnis 10:1 (Puffer zu Lösungsmittel) und gut gemischt. Danach erfolgt gegebenenfalls die Zugabe eines geeigneten Emulgators oder Dispergators, wie z.B. Bile-Salzen, (z.B. in Mengen von 1 mg pro µg Carotinoid). Weiterhin ist die Zugabe von stabilisierenden Salzen, wie z.B. einer NaCl/CaCl₂-Lösung, von Vorteil. Die Suspension wird anschließend ausreichend, z.B. für 30 Minuten bei 37°C, inkubiert. Für die enzymatische Hydrolyse der Carotinoidester wird dann eine Lipaselösung (z.B. Lipase Typ7 von Candida rugosa (Sigma)) zugegeben und unter Schütteln bei 37°C inkubiert. Nach einer ersten Inkubationsphase, wie z.B. etwa 21 Stunden, erfolgt nochmals eine Zugabe von Lipase mit erneuter Inkubation bei 37°C, z.B. über einen Zeitraum von mindestens etwa 5 Stunden, bis zur im Wesentlichen quantitativen Hydrolyse der Ester. Die eingesetzte Gesamtmenge an Lipase beträgt z.B. etwa 500 bis 3000 U/µg Carotinoid. Anschließend werden die Carotinoide durch kräftiges Mischen (nach Zugabe von Na₂SO₄ (wasserfrei)) in ein organisches, mit Wasser im Wesentlichen nicht mischbares Lösungsmittel, wie z.B. Petrolether, extrahiert. Dieses Ausschütteln wird solange wiederholt, bis die organische Phase farblos bleibt. Die organischen Fraktionen werden vereinigt und das Lösungsmittel wird evaporiert. Freie Carotinoide werden anschließend analysiert.

### f) Allgemeine Durchführung einer Esterase-katalysierten Esterhydrolyse

Zerkleinertes Ausgangsmaterial, z.B. gemörsertes Petalenmaterial, wird mit einem organischen Lösungsmittel, z.B.100% Aceton, gegebenenfalls mehrmals über einen geeigneten Zeitraum unter Schütteln (z.B. dreimal, jeweils etwa 15 Minuten) extrahiert. Das Lösungsmittel wird evaporiert. Carotinoide werden anschließend in einem geeigneten Volumen eines organischen Lösungsmittels, wie z.B. Aceton, aufgenommen (Absorption bei 475 nm z.B. zwischen 0,75 und 1,25) und suspendiert, z.B. durch 5-minütige Behandlung im Ultraschall-Bad. Der Carotinoid-Extrakt wird mit in einem geeigneten Puffer (z.B. Tris-HCl-Puffer, 50 mM; pH 7,0) gemischt und inkubiert (z.B. 5-10 Minuten bei 37°C). Danach erfolgt die Zugabe von Esterase (z.B. einer Cholesterol-Esterase von *Pseudomonas* spec. oder einer Cholesterol-Esterase von Pseudomonas fluorescens). Nach einer ersten Inkubationsphase (z.B. nach 8-12 Stunden) wird nochmals Enzym zugegeben; die im Wesentlichen quantitative Hydrolyse der Ester erfolgt innerhalb von etwa 24 Stunden bei Inkubation bei 37°C. Die eingesetzte Gesamtmenge an Esterase beträgt z.B. etwa 10 bis 500 U/mg Carotinoid. Nach Zugabe von Salz z.B. Na₂SO₄ (wasserfrei) wird mit einem mit Wasser im Wesentlichen nicht mischbaren organischen Lösungsmittel, wie Petrolether, gut gemischt und zentrifugiert (z.B. 3 Minuten; 4500g). Diese Vorgang wird gegebenenfalls mehrmals wiederholt. Die organischen Phasen werden vereinigt und evaporiert. Die gebildetenn Carotinoide werden anschließend analysiert.

### g) Esterhydrolyse in organischen Lösungsmittel anstelle eines wässrigen Milieus

Esterhydrolysen in organischem Lössungsmittel können von Vorteil sein, da
- organische Lösungsmittel das thermodynamische Equilibrium verschieben können,
- einige Lipasen ihr katalytisches Zentrum nur in Gegenwart einer Lipidphase oder vermutlich in organischem Lösungsmittel exponieren,
- die Verwendung von Bile-Salzen im wässrige Milieu kostenintensiv ist,
- Lipasen in organischen Lösungsmitteln über lange Zeiträume (Wochen bis Monate) auch bei erhöhten Temperaturen von z.B. 60°C aktiv bleiben.

Es kann die erfindungsgemäße Esterhydrolyse z.B. mit einer immobilisierte Lipase B von Candida antarctica , NOVO 435 von Novo Nordisk, in 100% n-Heptan zur Hydrolyse von Carotinoidester sogar bei erhöhten Temperaturen bis 60°C eingesetzt werden.

Es werden im Wesentlichen quantitative Hydrolyseraten beobachtet.

Als weitere brauchbare organische Reaktionsmedien sind n-Butanol, Hexan, Isooctan, Toluol, Butylether und Isopropylether denkbar.

### h) Aufarbeitung der Reaktionsprodukte

Das nach dem erfindungsgemäßen Verfahren hergestellte Produkt, wie z.B. Astaxanthin, lässt sich vorteilhaft aus der wässrigen Reaktionslösung über Extraktion gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether, Benzol, MTBE, Petrolether oder Essigester, ohne darauf beschränkt zu sein.

Wird dir Reaktion im organischen Medium durchgeführt, so ist in einem ersten Schritt die Abtrennung des Enzyms von der organischen Phase ausreichend.

Nach Einengen der so erhaltenen organischen Phase können die Produkte in der Regel in guten chemischen Reinheiten gewonnen werden.

Nach der Extraktion kann die organische Phase mit dem Produkt aber auch nur zum Teil eingeengt und das Produkt auskristallisiert werden. Dazu wird die Lösung vorteilhaft auf eine Temperatur von 0 °C bis 10°C abgekühlt. Die Kristallisation kann auch direkt aus der organischen Lösung erfolgen. Das auskristallisierte Produkt kann nochmals im gleichen oder in einem anderen Lösungsmittel zur erneuten Kristallisation aufgenommen werden und nochmals kristallisiert werden.

Es ist aber auch möglich das erfindungsgemäß hergestellte Produkt weiter aufzureinigen. Hierzu wird das produkthaltige Medium, gegebenenfalls nach dem Abtrennen des Enzyms, einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert.

Die Identität und Reinheit der isolierten Verbindung(en) kann durch bekannte Techniken bestimmt werden. Diese umfassen Hochleistungs-Flüssigkeitschromatographie (HPLC), spektroskopische Verfahren, Färbeverfahren, Dünnschichtchromatographie, Enzymtest oder mikrobiologische Tests. Diese Analyseverfahren sind zusammengefasst in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### i) Anwendungen der erfindungsgemäß hergestellten Produkte:

Die erfindungsgemäßen Hydrolyseprodukte eignen sich insbesondere als Nahrungs- und Futtermittelzusatz. Sie fördern vor allem die Pigmentierung nach, vorzugsweise oraler, Verabreichung.

Unter "Pigmentierung" wird erfindungsgemäß vorzugsweise die Intensivierung oder Verursachung einer Farbe zumindest eines Teils eines Tieres oder Tierproduktes des pigmentierten Tieres im Vergleich zum nicht pigmentierten Tier verstanden. So bewirken insbesondere Astaxanthin-haltige Pigmentierstoffe die Erzeugung oder Intensivierung eines rosa bis rosa-roten Farbtons.

Bevorzugte Tiere, die durch die erfindungsgemäße orale Verabreichung pigmentiert werden können, sind Tiere, ausgewählt unter Fischen, Crustaceaen oder Vögeln, insbesondere Galliformes und Anatidae. Bevorzugte Fische sind Salmoniden, insbesondere Lachs oder Forelle. Bevorzugte Crustaceae sind Shrimps oder Krebse. Bevorzugte Galliformes sind Hühner, Enten oder Gänse. Bevorzugter Anatidae ist Flamingo.

Je nach pigmentiertern Tier werden vorzugsweise unter pigmentierten Tierprodukten insbesondere Fleisch für Lachs oder Forelle, Haut für Hühner, Enten oder Gänse, Feder für Hühner, Enten, Gänse oder Flamingo und Ei bzw. Eidotter für Hühner, Enten oder Gänse verstanden.

Die orale Verabreichung der Carotinoide an Tiere kann direkt erfolgen oder, vorzugsweise, über orale Verabreichung von Tierfutterzubereitungen, denen zuvor das Carotinoid beigemischt wurden. Die Carotinoide können dabei in fester oder flüssiger Form vorliegen.

Die Carotinoide können, soweit die noch enthaltenen Lösungsmittel für die entsprechenden Tiere physiologisch unbedenklich sind, direkt der Tierfutterzubereitung zugegeben oder nach Abdampfen der noch enthaltenen Lösungsmittel in Form Carotinoid-haltiger Pulver oder Öle eingesetzt werden. Eine vorherige Aufreinigung des erhaltenen Hydrolyseproduktes ist nicht zwingend erforderlich.

Die erhaltenen Carotinoid-haltigen Pulver oder Öle können beispielsweise in Fischöl eingearbeitet werden, auf pulverige Trägermaterialien, wie beispielsweise Weizenmehl, aufgebracht werden, oder in Alginate, Gelatine oder Lipide eingeschlossen werden.

Der Erfindung betrifft daher auch Tierfutterzubereitungen, enthaltend neben üblichen Tierfutterbestandteilen wenigstens ein erfindungsgemäßes Carotinoid-Hydrolysat.

So kann beispielsweise eine Fischfutterzubereitung weitere übliche Fischfutterkomponenten enthalten, wie z.B. Fischmehl und/oder andere Proteine, Öle, wie beispielsweise Fischöle, Getreide, Vitamine, Mineralien, Konservierungsstoffe und gegebenenfalls Medikamente in üblichen Mengen.

Eine typische Fischfutterrezeptur für Forellen setzt sich beispielsweise aus folgenden Komponenten zusammen:

| | | **Einwaage f. 500 kg** |
|---|---|---|
| ***Komponenten*** | ***Gew.-%*** | ***kg*** |
| Fischmehl | 30,00 | 150,00 |
| Sojavollfettbohnen | 20,00 | 100,00 |
| Weizenquellstärke | 18,00 | 90,00 |
| Vitamin-Prämix | 0,80 | 4,00 |
| Cholinchlorid (50%) | 0,20 | 1,00 |
| Weizenkleber | 20,00 | 100,00 |
| Sipemat 50S | 3,00 | 15,00 |
| Fischöl | 8,00 | 40,00 |

Eine typische Fischfutterrezeptur für Lachse setzt sich beispielsweise aus folgenden Komponenten zusammen:

| | |
|---|---|
| ***Komponenten*** | ***Gew.-%*** |
| Fischmehl | 75,00 |
| Pflanzliches Protein | 5,00 |
| Getreide | 7,80 |
| Vitamine/Mineralien | 1,00 |
| Antioxidantien/Konservierungsstoffe | 0,20 |
| Fischöl | 11,00 |

Den Tierfutterzubereitungen können die erfindungsgemäßen Hydrolysate in Pulverform oder flüssig, wie z.B. als Öl beigemischt werden. Die so erhaltenen Tierfutterzubereitungen können in an sich bekannter Weise pelletiert oder besonders vorteilhaft extrudiert werden.

In einer bevorzugten Ausführungsform werden die Carotinoid-haltigen Präparate den Tierfutterzubereitungen vorzugsweise in flüssiger Form beigemischt. Dies ist insbesondere vorteilhaft bei der Herstellung von extrudierten Futterzubereitungen. Der Extrusionsprozess führt zu Extrusionsstress auf die empfindliche Stoffe, wie beispielsweise Astaxanthin, der zu einem Astaxanthinverlust führen kann. Bei Extrusionsstress handelt es sich primär um die Einwirkung mechanische Kräfte (Kneten, Scherung, Druck, etc.) jedoch auch um hydrothermischen Stress, verursacht durch Wasser- und Wasserdampfzugaben, auch oxidativer Stress ist zu beobachten.

Um die durch den oben beschriebenen Extrusionsprozess auftretende Stoffverluste zu vermeiden, können flüssige Carotinoid-haltige Extrakte durch die sogenannte PPA-Technik nach dem Extrusions - und Trocknungsprozess unter Vakuum appliziert werden (*post pelleting application*)*.*

Die Carotinoid-haltigen Hydrolysate können, soweit die noch enthaltenen Lösungsmittel für die entsprechenden Tiere physiologisch unbedenklich sind, auch direkt oral an Tiere verabreicht werden.

Die Carotinoid-haltigen Hydrolysate können aber auch erst nach Abdampfen der noch enthaltenen Lösungsmittel in Form von Pulver oder Ölen verabreicht werden.

Die erhaltenen Carotinoid-haltigen Pulver oder Öle können beispielsweise in Fischöl eingearbeitet werden, auf pulverige Trägermaterialien, wie beispielsweise Weizenmehl, aufgebracht werden, oder in Alginate, Gelatine oder Lipide eingeschlossen werden.

Der Erfindung betrifft daher auch Pigmentiermittel, enthaltend Carotinoid-haltigen Hydrolysate, wobei diese gegebenenfalls wie vorstehend beschrieben prozessiert sein können.

### Experimenteller Teil

### Beispiel 1: Enzymatische Cholesterol-Esterase-katalysierte Hydrolyse von Carotinoidestern aus Pflanzenmaterial und Identifizierung der Carotinoide

### Allgemeine Arbeitsvorschrift

Gemörsertes Pflanzenmaterial (z.B. Petalenmaterial) (50 bis 100 mg Frischgewicht) wird mit 100 % Aceton (dreimal 500 µl; jeweils etwa 15 Minuten schütteln) extrahiert. Das Lösungsmittel wird evaporiert. Carotinoide werden anschließend in 400 µl Aceton aufgenommen (Absorption bei 475 nm zwischen 0.75 und 1,25) und 5 min im Ultraschall-Bad behandelt. Der Carotinoid-Extrakt wird mit 300 µl 50 mM Tris-HCl-Puffer (pH 7,0) gemischt und 5 bis 10 Minuten bei 37°C inkubiert. Danach erfolgt die Zugabe von 100 bis 200 µl Cholesterol-Esterase (Stammlösung: 6,8 U/ml einer Cholesterol-Esterase von Pseudomonas spec.). Nach 8 bis 12 Stunden wird nochmals 100 bis 200 µl Enzym zugegeben; Hydrolyse der Ester erfolgt innerhalb von 24 Stunden bei Inkubation bei 37°C. Nach Zugabe von 0.35 g Na₂SO₄x10H₂0 und 500 µl Petrolether wird gut gemischt und zentrifugiert (3 Minuten; 4500 g). Petrolether-Phase wird abgezogen und nochmals mit 0,35 g Na₂SO₄ (anhydrous) gemischt. Zentrifugation für 1 Minute bei 10000 g. Petrolether wird evaporiert und freie Carotinoide werden in 100 bis 120 µl Aceton aufgenommen. Mittels HPLC und C30-reverse phase-Säule können freie Carotinoide aufgrund von Retentionszeit und UV-VIS-Spektren identifiziert werden.

### Beispiel 2: Enzymatische Lipase-katalysierte Hydrolyse von Carotinoidestern aus Pflanzenmaterial und Identifizierung der Carotinoide

### Allgemeine Arbeitsvorschrift

a) Gemörsertes Pflanzenmaterial (z.B. Petalenmaterial) (30-100 mg Frischgewicht) wird mit 100% Aceton (dreimal 500µl; jeweils etwa 15 Minuten schütteln) extrahiert. Das Lösungsmittel wird evaporiert. Carotinoide werden anschließend in 495 µl Aceton aufgenommen. 4,95 ml Kaliumphosphatpuffer (100 mM, pH7.4) zugegeben und gut gemischt. Danach erfolgt die Zugabe von ca. 17 mg Bile-Salze (Sigma) und 149 µl einer NaCl/CaCl₂-Lösung (3M NaCl und 75 mM CaCl₂). Die Suspension wird für 30 Minuten bei 37°C inkubiert. Für die enzymatische Hydrolyse der Carotinoidester wird 595 µl einer Lipaselösung (50 mg/ml Lipase Typ7 von Candida rugosa (Sigma)) zugegeben und unter Schütteln bei 37°C inkubiert. Nach etwa 21 Stunden erfolgte nochmals eine Zugabe von 595 µl Lipase mit erneuter Inkubation von mindestens 5 Stunden bei 37°C. Anschließend werden etwa 700 mg Na₂SO₄ (wasserfrei) in der Lösung gelöst. Nach Zugabe von 1800 µl Petrolether werden die Carotinoide durch kräftig Mischen in die organische Phase extrahiert. Dieses Ausschütteln wird solange wiederholt, bis die organische Phase farblos bleibt. Die Petroletherfraktionen werden vereinigt und der Petrolether evaporiert. Freie Carotinoide werden in 100-120 µl Aceton aufgenommen. Mittels HPLC und C30-reverse phase-Säule können freie Carotinoide aufgrund von Retentionszeit und UV-VIS-Spektren identifiziert werden.
   Die verwendeten Bile-Salze oder Gallensäuresalze sind 1:1 Mischungen von Cholat und Desoxycholat.
b) Arbeitsvorschrift für Aufarbeitung, wenn nur geringe Mengen an Carotinoidestem im Pflanzenmaterial vorhanden sind

Alternativ kann die Hydrolyse der Carotinoidester durch Lipase aus *Candida rugosa* nach Trennung mittels Dünnschichtchromatographie erreicht werden. Dazu werden 50-100mg Pflanzenmaterial dreimal mit etwa 750µl Aceton extrahiert. Der Lösungsmittelextrakt wird im Vakuum einrotiert (erhöhte Temperaturen von 40-50°C sind tolerabel). Danach erfolgt Zugabe von 300µl Petrolether:Aceton (Verhältnis 5:1) und gute Durchmischung. Schwebstoffe werden durch Zentrifugation (1-2 Minuten) sedimentiert. Die obere Phase wird in ein neues Reaktionsgefäß überführt. Das verbleibende Rest wird erneut mit 200µl Petrolether.Aceton (Verhältnis 5:1) extrahiert und Schwebstoffe werden durch Zentrifugation entfernt. Die beiden Extrakte werden zusammengeführt (Volumen 500µl) und die Lösungsmittel evaporiert. Der Rückstand wird in 30µl Petrolether:Aceton (Verhältnis 5:1) resuspendiert und auf eine Dünnschichtplatte (Silica-Gel 60, Merck) aufgetragen. Falls mehr als eine Auftragung für präparativ-analytische Zwecke erforderlich ist, sollten mehrere Aliquots mit jeweils 50-100 mg Frischgewicht in der beschriebenen Weise für die dünnschichtchromatographische Trennung aufbereitet werden.

Die Dünnschichtplatte wird in Petrolether:Aceton (Verhältnis 5:1) entwickelt. Carotinoidbanden können visuell aufgrund ihrer Farbe identifiziert werden. Einzelne Carotinoidbanden werden ausgekratzt und können für präparativ-analytische Zwecke gepoolt werden. Mit Aceton werden die Carotinoide vom Silica-Material eluiert; das Lösungsmittel wird im Vakuum evaporiert. Zur Hydrolyse der Carotinoidester wird der Rückstand in 495µl Aceton gelöst, 17mg Bile-Salze (Sigma), 4,95ml 0.1M Kaliumphosphatpuffer (pH 7,4) und 149µl 3M NaCl, 75mM CaCl₂ zugegeben. Nach guter Durchmischung wird 30min bei 37°C äquilibriert. Danach erfolgt die Zugabe von 595µl Lipase von Candida rugosa (Sigma, Stammlösung von 50mg/ml in 5mM CaCl₂). Ober Nacht erfolgt die Inkubation mit Lipase unter Schütteln bei 37°C. Nach etwa 21 Stunden wird nochmals die gleiche Menge an Lipase zugegeben; für mindestens 5 Stunden wird M/44120-PCT nochmals bei 37°C unter Schütteln inkubiert. Dann erfolgt die Zugabe von 700mg Na₂SO₄ (wasserfrei); mit 1800µl Petrolether wird für ca. 1 Minute ausgeschüttelt und die Mischung bei 3500 Umdrehungen/Minute für 5 Minuten zentrifugiert. Die obere Phase wird in ein neues Reaktionsgefäß überführt und das Ausschütteln so lange wiederholt, bis die obere Phase farblos ist. Die vereinigte Petrolether Phase wird im Vakuum eingeengt (Temperaturen von 40-50°C sind möglich). Der Rückstand wird in 120µl Aceton, eventuell mittels Ultraschall, gelöst. Die gelösten Carotinoide können mittels HPLC unter Verwendung einer C30-Säule getrennt und anhand von Referenzsubstanzen quantifiziert werden.

Obige Angaben in den Beispielen 1 und 2 sind entsprechend auf Algenmaterial übertragbar.

### Beispiel 3: HPLC-Analvse freier Carotinoide

Die Analyse der nach der Arbeitsvorschrift von Beispiel 1 und 2 erhaltenen Proben erfolgt unter folgenden Bedingungen:

Folgende HPLG-Bedingungen wurden eingestellt.
- Trennsäule:: Prontosil C30-Säule, 250 x 4,6 mm, (Bischoff, Leonberg, Germany)
- Flussrate:: 1.0 ml/min
- Eluenten:: Laufmittel A - 100% Methanol
Laufmittel B - 80% Methanol, 0.2% Ammoniumacetat
Laufmittel C - 100% t-Butyl-methylether
- Detektion:: 300-530 nm

### Gradientenprofil:

| Zeit | Flussrate | % Laufmittel A | % Laufmittel B | % Laufmittel C |
|---|---|---|---|---|
| 1.00 | 1.0 | 95.0 | 5.0 | 0 |
| 12.00 | 1.0 | 95.0 | 5.0 | 0 |
| 12.10 | 1.0 | 80.0 | 5.0 | 15.0 |
| 22.00 | 1.0 | 76.0 | 5.0 | 19.0 |
| 22.10 | 1.0 | 66.5 | 5.0 | 28.5 |
| 38.00 | 1.0 | 15.0 | 5.0 | 80.0 |
| 45.00 | 1.0 | 95.0 | 5.0 | 0 |
| 46.0 | 1.0 | 95.0 | 5.0 | 0 |

Einige typische Retentionszeiten für erfindungsgemäß gebildete Carotinoide sind z.B.: Violaxanthin 11, 7 min, Astaxanthin 17,7 min, Adonixanthin 19 min, Adonirubin 19,9 min, Zeaxanthin 21 min.

Einige typische Retentionszeiten-Bereiche für erfindungsgemäß gebildete Carotinoideester sind:

Bei Haematococcus sind die Carotinoide am häufigsten mit Palmitat, Oleat, Linolate und Linoleat verestert. Typische Retentionszeiten liegen bei 28 bis 40 Minuten.

Bei Tagetes sind die häufigsten Carotinoidester Dipalmitat. Myristat-Palmitat, Palmitat-Stearat und Dimyristat vertreten; die Monoester sind deutlich seltener. Typische Retenstionszeiten liegen bei 28 bis 38 Minuten.

Die Carotinoidester in Paprika tragen Fettsäuren der Längen C₁₂ bis C₁₆ wie z.B. Laurinsäure, Myristinsäure und Palmitinsäure. Am häufigsten kommen in rotem Paprika Carotinoide verestert mit Linolsäure, Palmitinsäure und Linolensäure vor. Typische Retenstionszeiten liegen bei 28 bis 40 Minuten.

Bei Adonis sind die Carotinoide am häufigsten mit Oleat, Palmitat, Myristat, Linoleat und Laurat verestert. Typischen Retentionszeiten liegen bei 28 bis 38 Minuten.

### Beispiel 4: Lipase-katalysierte Esterhydrolyse mit verschiedenen Carotinoidester-Quellen

Unter Befolgung der allgemeinen Lehre von Beispiel 2a werden Carotinoidester-Extrakte aus folgenden pflanzlichen Quellen bzw. Algen hergestellt und hydrolysiert:
Paprika (Capsicum annuum I.)
Adonis (Adonis aestivalis)
Tagetes (Tagetes erecta)
BioAstin (Haematococcus pluvialis)

Außerdem wurde der Emulgatorgehalt (Gehalt an Bile Salzen) bei verschiedenen Experimenten variiert.

Die ermittelten Hydrolyseraten sind in folgender Tabelle zusammengefasst:

### Hydrolyseraten für verschiedene Esterquellen

| Quelle | Lipase [U/µg Carotinoid] | Emulgator [mg/mg Carotinoid] | Hydrolyserate [%] | Abnahme der Hydrolyserate um [%] |
|---|---|---|---|---|
| Paprika | 2400 | 900 | 89 | - |
| | | 450 = [50%] | | 5 |
| Tagetes | 2700 | 800 | 95 | - |
| | | 560 = [70%] | | 7,4 |
| | | 400 = [50%] | | 15 |
| | | 200 = [25%] | | 10 |
| | | ohne | | 11,5 |
| Adonis | 700 | ohne | 96 bis 99 | - |
| BioAstin | 2200 | 800 | 95 | - |

| | | | | |
|---|---|---|---|---|
| Spezifische Aktivität der eingesetzten Lipase aus Candida rugosa: 819 U/mg Protein. | | | | |

Die Hydrolyseraten sind bezogen auf die Gesamtmenge an Ester. Dies sind bei Adonis und Haematococcus fast ausschließlich Ketocarotinoidester, bei Tagetes und Paprika ausschließlich Carotinoidester.

## Patentansprüche

1. Verfahren zur enzymatischen Hydrolyse von Ketocarotinoidestem, wobei man einen Ketocarotinoidester-haltigen Reaktanden, abgeleitet von einem natürlichen oder genetisch verändertem Organismus mit einem esterspaltenden Enzym, ausgewählt unter Lipasen (E.C. 3.1.1.3), bis zu einer 85-100 %igen hydrolytischen Esterspaltung inkubiert.

2. Verfahren nach Anspruch 1, wobei man das (die) gebildete(n) Ketocarotinoid(e) aus dem Reaktionsansatz isoliert.

3. Verfahren nach einem der vorhergehenden Ansprüche, wobei die Reaktionszeit im Bereich von 1 bis 48 Stunden liegt.

4. Verfahren nach einem der vorhergehenden Ansprüche, wobei dem Reaktionsansatz Enzym in einer Gesamtmenge zugesetzt wird, so dass die Gesamtkonzentration an zugesetzter Lipase bezogen auf den Gesamtcarotinoid-Gehalt im Bereich von 50 bis 3.000 U/µg liegt.

5. Verfahren nach einem der vorhergehenden Ansprüche, wobei man einen Ketocarotinoidester-haltigen Reaktanden in einem wasserhaltigen Reaktionsmedium mit dem esterspaltenden Enzym inkubiert.

6. Verfahren nach Anspruch 5, wobei die Ketocarotinoidester in emulgierter Form im Medium vorliegen.

7. Verfahren nach Anspruch 6, wobei dem Reaktionsmedium wenigstens ein Emulgator in einer Menge zugesetzt wird, so dass das Mengenverhältnis von Emulgator zu Gesamtcarotinoid im Bereich von 500:1 bis 1000:1 liegt.

8. Verfahren nach einem der Ansprüche 6 und 7, wobei der Emulgator wenigstens eine Verbindung, ausgewählt unter Cholansäure und Derivaten davon sowie Gemischen dieser Verbindungen umfasst.

9. Verfahren nach einem der Ansprüche 6 bis 8, wobei der Emulgator eine Mischung von Cholsäure und Desoxycholsäure ist.

10. Verfahren nach einem der vorhergehenden Ansprüche, wobei der Reaktionsansatz einen pH-Wert im Bereich von etwa 6 bis 8 aufweist.

11. Verfahren nach einem der vorhergehenden Ansprüche, wobei das Reaktionsmedium ein wässrig-organisches Medium ist, welches ein organisches Lösungsmittel in einem Volumenanteil von 5 bis 95 Vol.-%, bezogen auf das Gesamtvolumen des Reaktionsansatzes, enthält.

12. Verfahren nach Anspruch 11, wobei das organischen Lösungsmittel ausgewählt ist unter Aceton, C₄-C₁₀-Alkanen, C₄-C₁₀-Alkanolen, Di-C₂-C₆-Alkylethern und aromatischen Lösungsmitteln und Gemischen davon.

13. Verfahren nach einem der Ansprüche 1 bis 3, wobei man den Ketocarotinoidester-haltigen Reaktanden in einem nichtwässrigen Reaktionsmedium, das organisches Lösungsmittel in einem Anteil von mehr als 95 Vol.-% enthält, mit dem esterspaltenden Enzym inkubiert.

14. Verfahren nach Anspruch 13, wobei das nichtwässrige Reaktionsmedium wenigstens ein organisches Lösungsmittel, ausgewählt unter C₄-C₁₀-Alkanen, C₄₋C₁₀-Alkanolen, Di-C₂-C₆-Alkylethern und aromatischen Lösungsmittel enthält.

15. Verfahren nach einem der vorhergehenden Ansprüche, wobei das Enzym eine Lipase aus Candida sp. ist.

16. Verfahren nach einem der vorhergehenden Ansprüche, wobei der Reaktand wenigstens einen Ketocarotinoid-Monoester oder -Diester einer C₁₀₋₂₄-, vorzugsweise C₁₂₋₂₀-Monocarbonsäure umfasst.

17. Verfahren nach einem der vorhergehenden Ansprüche, wobei der Reaktand abgeleitet ist aus natürlichen oder rekombinanten, pro- oder eukaryotischen Mikroorganismen oder Pflanzen oder Teilen davon.

18. Verfahren nach Anspruch 17, wobei der Reaktand durch Extraktion mit Hilfe eines organischen Lösungsmittels oder Gemischen von organischen Lösungsmitteln gewonnen wird.

19. Verfahren nach einem der vorhergehenden Ansprüche, wobei die Reaktion mehrstufig unter wiederholter Zugabe von Enzym erfolgt.

20. Verfahren nach einem der vorhergehenden Ansprüche, wobei das Enzym in trägergebundener Form eingesetzt wird.

21. Verfahren nach einem der vorhergehenden Ansprüche, wobei die Reaktionstemperatur im Bereich von etwa 20 bis 70 °C liegt.

22. Verfahren nach einem der vorhergehenden Ansprüche, wobei der Ketocarotinoidester-haltige Reaktand wenigstens einen Ketocarotinoidester oder Gemische von Carotinoidestem und Ketocarotinoidestem umfasst.

23. Verfahren nach einem der vorhergehenden Ansprüche, wobei man die gespaltenen Ketocarotinoide aus dem Reaktionsmedium isoliert.

24. Verfahren nach einem der vorhergehenden Ansprüche, wobei der Ketocarotinoidester abgeleitet ist von Astaxanthin.

25. Verwendung der nach einem der vorhergehenden Ansprüche hergestellten Ketocarotinoide zur Herstellung von Nahrungs- und Futtermittelzusätzen.

## Claims

1. A process for the enzymatic hydrolysis of ketocarotenoid esters, where a ketocarotenoid ester-containing reactant derived from a natural or genetically modified organism is incubated with an ester-cleaving enzyme selected from lipases (E.C. 3.1.1.3) until an at least approximately 85-100% hydrolytic ester cleavage.

2. A process as claimed in claim 1, where the ketocarotenoid(s) which is(are) formed is(are) isolated from the reaction mixture.

3. A process as claimed in any of the preceding claims, where the reaction time is in the range of 1 to 48 hours.

4. A process as claimed in any of the preceding claims, where the total amount of enzyme added to the reaction mixture is such that the total concentration of added lipase based on the total carotenoid content is in the range of 50 to 3,000 U/µg.

5. A process as claimed in any of the preceding claims, where a ketocarotenoid ester-containing reactant is incubated in an aqueous reaction medium with the ester-cleaving enzyme.

6. A process as claimed in claim 5, where the ketocarotenoid esters are present in emulsified form in the medium.

7. A process as claimed in claim 6, where at least one emulsifier is added to the reaction medium in an amount such that the ratio of the amounts of emulsifier to total carotenoid is in the range of 500:1 to 1000:1.

8. A process as claimed in claims 6 and 7, where the emulsifier includes at least one compound selected from cholanic acid and derivatives thereof, and mixtures of these compounds.

9. A process as claimed in any of claims 6 to 8, where the emulsifier is a mixture of cholic acid and deoxycholic acid.

10. A process as claimed in any of the preceding claims, where the reaction mixture has a pH in the range of about 6 to 8.

11. A process as claimed in any of the preceding claims, where the reaction medium is an aqueous/organic medium which comprises an organic solvent in a proportion by volume of 5 to 95% by volume based on the total volume of reaction mixture.

12. A process as claimed in claim 11, where the organic solvent is selected from acetone, C₄-C₁₀-alkanes, C₄-C₁₀-alkanols, di-C₂-C₆-alkyl ethers and aromatic solvents and mixtures thereof.

13. A process as claimed in claims 1 to 3, where the ketocarotenoid ester-containing reactant is incubated with the ester-cleaving enzyme in a nonaqueous reaction medium comprising an organic solvent in a proportion of more than 95% by volume.

14. A process as claimed in claim 13, where the nonaqueous reaction medium comprises at least one organic solvent selected from C₄-C₁₀-alkanes, C₄-C₁₀₋alkanols, di-C₂-C₆-alkyl ethers and aromatic solvents.

15. A process as claimed in any of the preceding claims, where the enzyme is a lipase from Candida sp..

16. A process as claimed in any of the preceding claims, where the reactant comprises at least one ketocarotenoid monoester or diester of a C₁₀₋₂₄, preferably C₁₂₋₂₀ monocarboxylic acid.

17. A process as claimed in any of the preceding claims, where the reactant is derived from natural or recombinant pro- or eukaryotic microorganisms or plants or parts thereof.

18. A process as claimed in claim 17, where the reactant is obtained by extraction with the aid of an organic solvent or mixtures of organic solvents.

19. A process as claimed in any of the preceding claims, where the reaction is carried out in a plurality of stages with repeated addition of enzyme.

20. A process as claimed in any of the preceding claims, where the enzyme is employed in carrier-bound form.

21. A process as claimed in any of the preceding claims, where the reaction temperature is in the range of about 20 to 70°C.

22. A process as claimed in any of the preceding claims, where the ketocarotenoid ester-containing reactant includes at least one ketocarotenoid ester or mixtures of carotenoid esters and ketocarotenoid esters.

23. A process as claimed in any of the preceding claims, where the cleaved ketocarotenoids are isolated from the reaction medium.

24. A process as claimed in any of the preceding claims, where the ketocarotenoid ester is derived from astaxanthin.

25. The use of the ketocarotenoids prepared as set forth in any of the preceding claims for producing human and animal food additions.

## Revendications

1. Procédé d'hydrolyse enzymatique d'esters de cétocarotinoïde, dans lequel on met à incuber un réactif contenant de l'ester de cétocarotinoïde, dérivé d'un organisme naturel ou génétiquement modifié, avec une enzyme saponifiante choisie parmi des lipases (E.C. 3.1.1.3) jusqu'à une saponification par hydrolyse à 85-100 %.

2. Procédé suivant la revendication 1, dans lequel on isole de la masse réactionnelle le ou les cétocarotinoïdes formés.

3. Procédé suivant l'une des revendications précédentes, dans lequel le temps de réaction est de l'ordre de 1 à 48 heures.

4. Procédé suivant l'une des revendications précédentes, dans lequel on ajoute de l'enzyme à la masse réactionnelle en une quantité totale telle que la concentration globale en lipase ajoutée soit, par rapport à la teneur totale en carotinoïde, de l'ordre de 50 à 3 000 U/µg.

5. Procédé suivant l'une des revendications précédentes, dans lequel on met à incuber avec l'enzyme saponifiante un réactif contenant de l'ester de cétocarotinoïde dans un milieu réactionnel contenant de l'eau.

6. Procédé suivant la revendication 5, dans lequel les esters de cétocarotinoïde se présentent sous forme émulsionnée dans le milieu.

7. Procédé suivant la revendication 6, dans lequel on ajoute au milieu réactionnel au moins un agent émulsionnant en une quantité telle que le rapport quantitatif entre l'agent émulsionnant et le carotinoïde total soit de l'ordre de 500/1 à 1 000/1.

8. Procédé suivant l'une des revendications 6 et 7, dans lequel l'agent émulsionnant comprend au moins un composé choisi parmi de l'acide cholanique et ses dérivés ainsi que des mélanges de ces composés.

9. Procédé suivant l'une des revendications 6 à 8, dans lequel l'agent émulsionnant est un mélange d'acide cholique et d'acide désoxycholique.

10. Procédé suivant l'une des revendications précédentes, dans lequel la masse réactionnelle présente une valeur de pH de l'ordre d'environ 6 à 8.

11. Procédé suivant l'une des revendications précédentes, dans lequel le milieu réactionnel est un milieu organique-aqueux qui contient un solvant organique en une fraction volumique de 5 à 95 % en volume, par rapport au volume total de la masse réactionnelle.

12. Procédé suivant la revendication 11, dans lequel le solvant organique est choisi parmi de l'acétone, des alcanes en C₄-C₁₀, des alcanols en C₄-C₁₀, des éthers dialkyliques en C₂-C₈ et des solvants aromatiques et leurs mélanges.

13. Procédé suivant l'une des revendications 1 à 3, dans lequel on met à incuber avec l'enzyme saponifiante le réactif contenant de l'ester de cétocarotinoïde dans un milieu réactionnel non aqueux qui contient du solvant organique en une fraction supérieure à 95 % en volume.

14. Procédé suivant la revendication 13, dans lequel le milieu réactionnel non aqueux contient au moins un solvant organique choisi parmi des alcanes en C₄-C₁₀, des alcanols en C₄-C₁₀, des éthers dialkyliques en C₂-C₈ et des solvants aromatiques.

15. Procédé suivant l'une des revendications précédentes, dans lequel l'enzyme est une lipase issue de Candida sp.

16. Procédé suivant l'une des revendications précédentes, dans lequel le réactif comporte au moins un monoester ou diester de cétocarotinoïde d'un acide monocarboxylique en C₁₀-C₂₄, de préférence en C₁₂-C₂₀.

17. Procédé suivant l'une des revendications précédentes, dans lequel le réactif est dérivé de micro-organismes procaryotes ou eucaryotes, naturels ou recombinants, ou de plantes ou de parties de celles-ci.

18. Procédé suivant la revendication 17, dans lequel le réactif est obtenu par extraction à l'aide d'un solvant organique ou de mélanges de solvants organiques.

19. Procédé suivant l'une des revendications précédentes, dans lequel la réaction a lieu en plusieurs étapes, avec addition répétée d'enzyme.

20. Procédé suivant l'une des revendications précédentes, dans lequel l'enzyme est mise en oeuvre sous une forme fixée sur un support.

21. Procédé suivant l'une des revendications précédentes, dans lequel la température réactionnelle est de l'ordre d'environ 20 à 70°C.

22. Procédé suivant l'une des revendications précédentes, dans lequel le réactif contenant de l'ester de cétocarotinoïde comporte au moins un ester de cétocarotinoïde ou des mélanges d'esters de carotinoïde et d'esters de cétocarotinoïde.

23. Procédé suivant l'une des revendications précédentes, dans lequel on isole du milieu réactionnel les cétocarotinoïdes séparés.

24. Procédé suivant l'une des revendications précédentes, dans lequel l'ester de cétocarotinoïde est dérivé d'astaxanthine.

25. Utilisation des cétocarotinoïdes préparés suivant l'une des revendications précédentes, pour la préparation d'additifs nutritifs et d'additifs d'aliments pour animaux.
